(19) **Europäisches Patentamt** / **European Patent Office** / **Office européen des brevets**

(11) **EP 4 223 693 A1**

(12) **EUROPEAN PATENT APPLICATION**
published in accordance with Art. 153(4) EPC

(43) Date of publication:
**09.08.2023 Bulletin 2023/32**

(21) Application number: **21875414.1**

(22) Date of filing: **24.09.2021**

(51) International Patent Classification (IPC):
*C01B 32/306* [(2017.01)]     *B01J 20/20* [(2006.01)]
*B01J 20/28* [(2006.01)]     *G01N 30/88* [(2006.01)]

(52) Cooperative Patent Classification (CPC):
**B01J 20/20; B01J 20/28; C01B 32/306; G01N 30/88**

(86) International application number:
**PCT/JP2021/035076**

(87) International publication number:
**WO 2022/071112 (07.04.2022 Gazette 2022/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.09.2020 JP 2020165322**

(71) Applicants:
• **Futamura Kagaku Kabushiki Kaisha**
  **Nakamura-ku**
  **Nagoya-shi**
  **Aichi 450-0002 (JP)**
• **National Institute Of Advanced Industrial Science and Technology**
  **Chiyoda-ku**
  **Tokyo 100-8921 (JP)**

(72) Inventors:
• **YAMASHITA Nobuyoshi**
  **Tsukuba-shi Ibaraki 305-8560 (JP)**
• **TANIYASU Sachi**
  **Tsukuba-shi Ibaraki 305-8560 (JP)**
• **KOUSAKA Tsutomu**
  **Nagoya-shi Aichi 450-0002 (JP)**
• **YOKOI Makoto**
  **Minokamo-shi Gifu 505-0024 (JP)**
• **HORI Chiharu**
  **Minokamo-shi Gifu 505-0024 (JP)**
• **SHIMAMURA Kodai**
  **Minokamo-shi Gifu 505-0024 (JP)**
• **ASANO Takuya**
  **Minokamo-shi Gifu 505-0024 (JP)**

(74) Representative: **TBK**
  **Bavariaring 4-6**
  **80336 München (DE)**

(54) **NEUTRAL PER- AND POLY-FLUOROALKYL COMPOUND ADSORBING ACTIVATED CARBON AND METHOD FOR ANALYZING NEUTRAL PER- AND POLY-FLUOROALKYL COMPOUNDS IN WATER SAMPLE**

(57)     [OBJECT] To provide an activated carbon for adsorbing neutral per- and polyfluoroalkyl compounds which is used in quantitative analysis of neutral per- and polyfluoroalkyl compounds in a water sample and which is capable of desorbably collecting neutral per- and polyfluoroalkyl compounds.

[ACHIEVING MEANS] An activated carbon for adsorbing neutral per- and polyfluoroalkyl compounds used in quantitative analysis of neutral per- and polyfluoroalkyl compounds in a water sample includes an activated carbon having a BET specific surface area of 900 $m^2$/g or more for desorbably adsorbing neutral per- and polyfluoroalkyl compounds distributed in a gas phase from the water sample by aerating or bubbling, with an inert gas as a carrier gas, the water sample sealed in a vessel and temperature-controlled to a temperature at which the neutral per- and polyfluoroalkyl compounds can be distributed in a gas phase.

EP 4 223 693 A1

Fig. 3

1

F

A

W

G

**Description**

FIELD

The present invention relates to an activated carbon used in quantitative analysis of neutral per- and polyfluoroalkyl compounds contained in a water sample and an analysis method.

BACKGROUND

Per- and polyfluoroalkyl compounds are fluorine-substituted aliphatic compounds having high thermal stability, high chemical stability, and high surface modification activity. Per- and polyfluoroalkyl compounds are widely used in industrial applications such as surface treatment agents, packaging materials, liquid fire-extinguishing agents, and chemical applications which take advantage of the characteristics described above.

Since some per- and polyfluoroalkyl compounds are highly stable chemical substances, they are not easily decomposed under natural conditions after being released into the environment. For this reason, in recent years, per- and polyfluoroalkyl compounds have been recognized as Persistent Organic Pollutants (POPs), and from 2010, the production and use of perfluorooctane sulfonic acid (PFOS) (IUPAC name: 1,1,2,2,3,3,4,4,5,5,6,6,7,7,8,8,8-heptadecafluorooctane-1-sulfonic acid) are regulated under the Stockholm Convention on Persistent Organic Pollutants (POPs Convention).

Note that perfluoroalkyl compounds have a completely fluorinated linear alkyl group and are substances represented by the chemical formula (ii). Examples thereof include perfluorooctane sulfonic acid (PFOS) and perfluorooctanoic acid (PFOA) (IUPAC name: 2,2,3,3,4,4,5,5,6,6,7,7,8,8,8-pentadecafluoroocanoic acid).

[Formula 2]

$$C_n F_{2n+1} \text{-} R \cdots \qquad \text{(ii)}$$

Polyfluoroalkyl compounds refer to compounds having an alkyl group in which some of the hydrogen atoms thereof have been replaced with fluorine atoms, and are substances represented by the chemical formula (iii). Examples thereof include fluorotelomer alcohols (FTOHs).

[Formula 3]

$$C_n F_{2n+1} \text{-} C_2 H_4 \text{-} R \cdots \qquad \text{(iii)}$$

In this manner, per- and polyfluoroalkyl compounds remain in the natural world (in water, soil, and the atmosphere), and thus, establishment of quantitative test methods for per- and polyfluoroalkyl compounds is being investigated. The challenge for studying quantitative test methods is the development of collection materials having high adsorption and desorption performance of per- and polyfluoroalkyl compounds. Water or air, as a sample containing a trace amount of per- and polyfluoroalkyl compounds, is brought into contact with a collection material to collect the per- and polyfluoroalkyl compounds, and the compounds adsorbed on the collection material are desorbed into an extraction liquid in an extraction step and concentrated. After concentration, quantitative measurement can be performed with a device such as an LC-MS/MS or GC-MS/MS to measure the concentration of per- and polyfluoroalkyl compounds contained in the sample.

As an existing collection material, for example, an organic fluorine-based compound-adsorbing material composed of a cyclodextrin polymer has been proposed (Patent Literature 1). This adsorbing material is not suitable for use as a collection material used for quantitative measurement because it is specialized only for adsorption and compounds cannot be desorbed therefrom. Furthermore, the cyclodextrin polymer is in the form of a powder or fine particles, and has problems such as poor handleability, high resistance during liquid passage or aeration, and a risk of outflow of the fine powder to the secondary side.

Furthermore, per- and polyfluoroalkyl compounds remain in the environment in various forms having a wide range of physicochemical properties, and existing adsorption materials do not have sufficient collection performance, whereby there is a problem in that accurate quantitative measurement cannot be performed.

As a method for analyzing, among these per- and polyfluoroalkyl compounds, ionic per- and polyfluoroalkyl compounds such as PFOS and PFOA, solid-phase extraction is mainly used. For water-soluble compounds such as ionic per- and polyfluoroalkyl compounds, analysis by solid-phase extraction is considered effective. However, since neutral per- and polyfluoroalkyl compounds such as FTOHs are poorly water-soluble and volatile, it is considered difficult to analyze them by solid-phase extraction.

The applicant has investigated to enable quantitative analysis of neutral per- and polyfluoroalkyl compounds, has investigated activated carbon as a collection material for neutral per- and polyfluoroalkyl compounds used in the analysis method to establish an analysis method for neutral per- and polyfluoroalkyl compounds in a water sample, and has discovered that it enables effective collection of neutral per- and polyfluoroalkyl compounds and greatly contributes to highly accurate quantitative measurement.

[CITATION LIST]

[PATENT LITERATURE]

[PTL 1] Japanese Unexamined Patent Publication (Kokai) No. 2012-101159

SUMMARY

[TECHNICAL PROBLEM]

The present invention has been conceived of in light of the points above, and in particular, provides an activated carbon for adsorbing neutral per- and polyfluoroalkyl compounds, which is used in quantitative analysis of neutral per- and polyfluoroalkyl compounds in a water sample and which is capable of desorbably collecting neutral per- and polyfluoroalkyl compounds, and an analysis method for neutral per- and polyfluoroalkyl compounds in a water sample.

[SOLUTION TO PROBLEM]

Specifically, a first invention relates to an activated carbon for adsorbing neutral per- and polyfluoroalkyl compounds for use in quantitative analysis of neutral per- and polyfluoroalkyl compounds in a water sample, comprising an activated carbon having a BET specific surface area of 900 $m^2$/g or more for desorbably adsorbing neutral per- and polyfluoroalkyl compounds distributed in a gas phase from the water sample by aerating or bubbling, with an inert gas as a carrier gas, the water sample temperature-controlled to a temperature at which neutral per- and polyfluoroalkyl compounds can be distributed in a gas phase.

A second invention relates to the activated carbon for adsorbing neutral per- and polyfluoroalkyl compounds of the first invention, wherein a sum ($V_{mic}$) of a volume of micropores of 1 nm or less of the activated carbon is 0.35 $cm^3$/g or more.

A third invention relates to the activated carbon for adsorbing neutral per- and polyfluoroalkyl compounds of the first or second invention, wherein a sum ($V_{met}$) of a volume of mesopores of 2 to 60 nm or less of the activated carbon is 0.02 $cm^3$/g or more.

A fourth invention relates to the activated carbon for adsorbing neutral per- and polyfluoroalkyl compounds of any of the first through third inventions, wherein a volume difference ($V_s$) between the sum ($V_{mic}$) of the volume of micropores and the sum ($V_{met}$) of the volume of mesopores of the activated carbon as prescribed by the following formula (i) is 0.45 $cm^3$/g or more.

[Formula 1]

$$V_s = V_{mic} - V_{met} \quad \cdots \text{( i )}$$

A fifth invention relates to the activated carbon for adsorbing neutral per- and polyfluoroalkyl compounds of any of the first through fourth inventions, wherein a surface oxide amount of the activated carbon is 0.10 meq/g or more.

A sixth invention relates to a filter body for adsorbing neutral per-and polyfluoroalkyl compounds, comprising a disk-shaped solid phase column filled with the activated carbon for adsorbing neutral per- and polyfluoroalkyl compounds in a water sample of any of the first through fifth inventions.

A seventh invention relates to an analysis method for quantitative analysis of neutral per- and polyfluoroalkyl compounds in a water sample, the method comprising: aerating or bubbling, with an inert gas as a carrier gas, a water sample sealed in a vessel and temperature-controlled to a temperature at which neutral per- and polyfluoroalkyl compounds can be distributed in a gas phase, distributing the neutral per- and polyfluoroalkyl compounds in the gas phase from the water sample, and adsorbing the neutral per- and polyfluoroalkyl compounds with a solid phase adsorbent, and subjecting the neutral per- and polyfluoroalkyl compounds adsorbed by the solid phase adsorbent to extraction with an organic solvent and measurement.

An eighth invention relates to the analysis method for neutral per- and polyfluoroalkyl compounds in a water sample of the seventh invention, wherein the solid phase adsorbent is an activated carbon adsorbent.

A ninth invention relates to the analysis method for neutral per- and polyfluoroalkyl compounds in a water sample of the seventh or eighth invention, wherein the water sample is temperature-controlled to 25 to 60 °C.

A tenth invention relates to the analysis method for neutral per- and polyfluoroalkyl compounds in a water sample of any of the seventh through ninth inventions, wherein aerating or bubbling of the inert gas is performed for 10 to 60 minutes.

An eleventh invention relates to the analysis method for neutral per- and polyfluoroalkyl compounds in a water sample of any of the seventh through tenth inventions, wherein a main component of the organic solvent is a mixed solvent of dichloromethane and ethyl acetate.

A twelfth invention relates to the analysis method for neutral per- and polyfluoroalkyl compounds in a water sample of any of the eighth through eleventh inventions, wherein the BET specific surface area of the activated carbon is 900 $m^2$/g or more.

A thirteenth invention relates to the analysis method for neutral per- and polyfluoroalkyl compounds in a water sample of any of the eight through twelfth inventions, wherein a sum ($V_{mic}$) of a volume of micropores of 1 nm or less of the activated carbon is 0.35 $cm^3$/g or more.

A fourteenth invention relates to the analysis method for neutral per- and polyfluoroalkyl compounds in a water sample of any of the eighth through thirteenth inventions, wherein a sum ($V_{met}$) of a volume of mesopores of 2 to 60 nm or less of the activated carbon is 0.02 $cm^3$/g or more.

A fifteenth invention relates to the analysis method for neutral per- and polyfluoroalkyl compounds in a water sample of any of the eighth through fourteenth inventions, wherein a volume difference ($V_s$) between the sum ($V_{mic}$) of the volume of micropores and the sum ($V_{met}$) of the volume of mesopores of the activated carbon as prescribed by the following formula (i) is 0.45 $cm^3$/g or more.

A sixteenth invention relates to the analysis method for neutral per- and polyfluoroalkyl compounds in a water sample of any of the eighth through fifteenth inventions, wherein a surface oxide amount of the activated carbon is 0.10 meq/g or more.

[ADVANTAGEOUS EFFECTS OF INVENTION]

The activated carbon for adsorbing neutral per- and polyfluoroalkyl compounds according to the first invention is an activated carbon for use in quantitative analysis of neutral per- and polyfluoroalkyl compounds in a water sample, comprising an activated carbon having a BET specific surface area of 900 $m^2$/g or more for desorbably adsorbing neutral per- and polyfluoroalkyl compounds distributed in a gas phase from the water sample by aerating or bubbling, with an inert gas as a carrier gas, the water sample temperature-controlled to a temperature at which neutral per-and polyfluoroalkyl compounds can be distributed in a gas phase, whereby quantitative measurement of neutral per- and polyfluoroalkyl compounds in a water sample, has not been established at this time, can be performed, such that the compounds can be efficiently collected and the accuracy of quantitative analysis can be improved.

The activated carbon for adsorbing neutral per- and polyfluoroalkyl compounds according to the second invention is the first invention, wherein a sum ($V_{mic}$) of a volume of micropores of 1 nm or less of the activated carbon is 0.35 $cm^3$/g or more, whereby neutral per- and polyfluoroalkyl compounds can efficiently and detachably be collected and the accuracy of quantitative analysis of such compounds can be improved.

The activated carbon for adsorbing neutral per- and polyfluoroalkyl compounds according to the third invention is the first or second invention, wherein a sum ($V_{met}$) of a volume of mesopores of 2 to 60 nm or less of the activated carbon is 0.02 $cm^3$/g or more, whereby neutral per- and polyfluoroalkyl compounds can efficiently and detachably be collected and the accuracy of quantitative analysis of such compounds can be improved.

The activated carbon for adsorbing neutral per- and polyfluoroalkyl compounds according to the fourth invention is any of the first through third inventions, wherein a volume difference ($V_s$) between the sum ($V_{mic}$) of the volume of micropores and the sum ($V_{met}$) of the volume of mesopores of the activated carbon as prescribed by the following formula (i) is 0.45 $cm^3$/g or more, whereby neutral per- and polyfluoroalkyl compounds can efficiently and detachably be collected and the accuracy of quantitative analysis of such compounds can be improved.

The activated carbon for adsorbing neutral per- and polyfluoroalkyl compounds according to the fifth invention is any of the first through fourth inventions, wherein a surface oxide amount of the activated carbon is 0.10 meq/g or more, there is provided not only adsorption performance due to the pores of the activated carbon, but also chemical adsorption capability, whereby the neutral per- and polyfluoroalkyl compound adsorption performance can further be improved and the accuracy of quantitative analysis of such compounds can be improved.

The filter body for adsorbing neutral per- and polyfluoroalkyl compounds according to the sixth invention comprises a disk-shaped solid phase column filled with the activated carbon for adsorbing neutral per- and polyfluoroalkyl compounds in a water sample of any of the first through fifth inventions, whereby suitable handling properties can be provided while increasing the neutral per- and polyfluoroalkyl compound collection efficiency.

The analysis method for neutral per- and polyfluoroalkyl compounds in a water sample according to the seventh invention is an analysis method for quantitative analysis of neutral per-and polyfluoroalkyl compounds in a water sample, the method comprising: aerating or bubbling, with an inert gas as a carrier gas, a water sample sealed in a vessel and temperature-controlled to a temperature at which neutral per- and polyfluoroalkyl compounds can be distributed in a gas phase, distributing the neutral per- and polyfluoroalkyl compounds in the gas phase from the water sample, and adsorbing the neutral per- and polyfluoroalkyl compounds with a solid phase adsorbent, and subjecting the neutral per- and polyfluoroalkyl compounds adsorbed by the solid phase adsorbent to extraction with an organic solvent and measurement, whereby quantitative measurement of neutral per- and polyfluoroalkyl compounds in a water sample, which has not been established at this time, can be made possible, whereby the accuracy of quantitative analysis can be improved.

The analysis method for neutral per- and polyfluoroalkyl compounds in a water sample according to the eighth invention is the seventh invention, wherein the solid phase adsorbent is an activated carbon adsorbent, whereby quantitative measurement of neutral per- and polyfluoroalkyl compounds in a water sample can be performed with higher accuracy.

The analysis method for neutral per- and polyfluoroalkyl compounds in a water sample according to the ninth invention is the seventh or eighth invention, wherein the water sample is temperature-controlled to 25 to 60 °C, whereby quantitative measurement of neutral per- and polyfluoroalkyl compounds in a water sample can be efficiently performed.

The analysis method for neutral per- and polyfluoroalkyl compounds in a water sample according to the tenth invention is any one of the seventh through ninth inventions, wherein aerating or bubbling of the inert gas is performed for 10 to 60 minutes, whereby quantitative measurement of neutral per- and polyfluoroalkyl compounds in a water sample can be efficiently performed.

The analysis method for neutral per- and polyfluoroalkyl compounds in a water sample according to the eleventh invention is any of the seventh through tenth inventions, wherein a main component of the organic solvent is a mixed solvent of dichloromethane and ethyl acetate, whereby such compounds can be successfully extracted from the solid phase adsorbent.

The analysis method for neutral per- and polyfluoroalkyl compounds in a water sample according to the twelfth is any of the eighth through eleventh inventions, wherein the BET specific surface area of the activated carbon is 900 $m^2/g$ or more, whereby neutral per- and polyfluoroalkyl compounds can suitably be desorbed.

The analysis method for neutral per- and polyfluoroalkyl compounds in a water sample according to the thirteenth invention is any of the eighth through eleventh inventions, wherein a sum ($V_{mic}$) of a volume of micropores of 1 nm or less of the activated carbon is 0.35 $cm^3/g$ or more, whereby neutral per- and polyfluoroalkyl compounds can efficiently and detachably be collected and the accuracy of quantitative analysis of such compounds can be improved.

The analysis method for neutral per- and polyfluoroalkyl compounds in a water sample according to the fourteenth invention is any of the eighth through thirteenth inventions, wherein a sum ($V_{met}$) of a volume of mesopores of 2 to 60 nm or less of the activated carbon is 0.02 $cm^3/g$ or more, whereby neutral per- and polyfluoroalkyl compounds can efficiently and detachably be collected, and the accuracy of quantitative analysis of such compounds can be improved.

The analysis method for neutral per- and polyfluoroalkyl compounds in a water sample according to the fifteenth invention is any of the eighth through fourteenth inventions, wherein a volume difference ($V_s$) between the sum ($V_{mic}$) of the volume of micropores and the sum ($V_{met}$) of the volume of mesopores of the activated carbon as prescribed by the following formula (i) is 0.45 $cm^3/g$ or more, whereby neutral per- and polyfluoroalkyl compounds can efficiently and detachably be collected, and the accuracy of quantitative analysis of such compounds can be improved.

The analysis method for neutral per- and polyfluoroalkyl compounds in a water sample according to the sixteenth invention is any of the eighth through fifteenth inventions, wherein a surface oxide amount of the activated carbon is 0.10 meq/g or more, whereby there is provided not only adsorption performance due to the pores of the activated carbon, but also chemical adsorption capability, the neutral per- and polyfluoroalkyl compound adsorption performance can further be improved, and the accuracy of quantitative analysis of such compounds can be improved.

BRIEF DESCRIPTION OF DRAWINGS

FIG. 1 is a schematic view showing a disk-type solid phase column filled with the activated carbon for adsorbing neutral per- and polyfluoroalkyl compounds of the present invention.

FIG. 2 is a partial cross-sectional view of the disk-type solid phase column of FIG. 1.

FIG. 3 is a conceptual diagram of a purge and trap method using the activated carbon for adsorbing neutral per- and polyfluoroalkyl compounds of the present invention as a solid phase adsorbent.

DESCRIPTION OF EMBODIMENTS

The analysis method of the present invention is an analysis method which enables quantitative measurement of neutral per- and polyfluoroalkyl compounds, which are poorly water-soluble and volatile, present in a water sample. Furthermore, the activated carbon for adsorbing neutral per-and polyfluoroalkyl compounds of the present invention is an activated carbon for use in an analysis method which enables quantitative measurement of neutral per- and polyfluoroalkyl compounds, which are poorly water-soluble and volatile, present in a water sample.

The measurement accuracy of the analysis method depends greatly on the neutral per- and polyfluoroalkyl compound adsorption performance of the activated carbon as the solid phase adsorbent used. Thus, it is necessary that the activated carbon for adsorbing neutral per- and polyfluoroalkyl compounds of the present invention have suitable adsorption performance for such compounds.

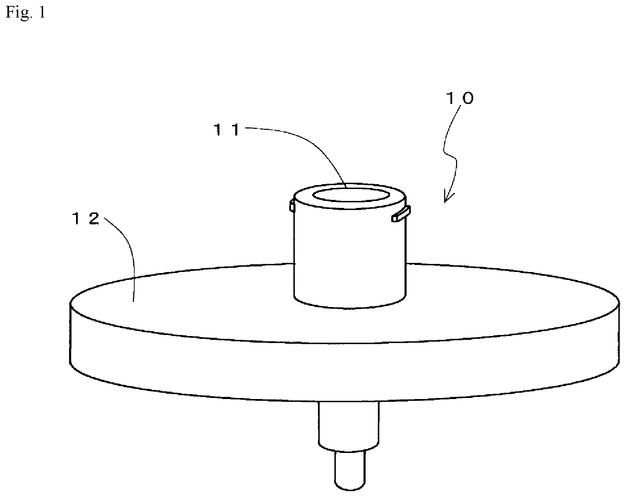

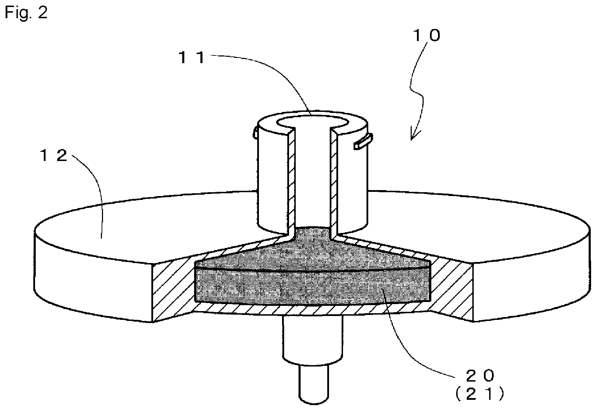

The activated carbon is preferably housed in a disk-type solid phase column 10 as shown in FIGS. 1 and 2. By housing the activated carbon filter part 20 in the solid phase column, analysis can be performed immediately after removing the solid phase column. In the disk-type solid phase column 10, it is considered that since the carrier gas containing the neutral per- and polyfluoroalkyl compounds distributed from the water sample into the gas phase spreads from the intake opening 11 to the body 12, adsorption efficiency is improved. The activated carbon filter part 20 can be easily handled if it is formed, for example, into a felt-like filter part 21 as shown in FIG. 2.

The activated carbon is composed of a fiber-like activated carbon or a granular activated carbon. The fiber-like activated carbon is an activated carbon obtained by carbonizing and activating appropriate fibers, and examples thereof include phenol resin type, acrylic resin type, cellulose type, and coal pitch type activated carbons. The fiber length, cross-sectional diameter, etc., are appropriate.

Examples of the raw material of granular activated carbons include raw materials such as wood (waste wood, wood from thinning, and sawdust), coffee bean pomace, rice husks, coconut husks, bark, and fruit nuts. These naturally derived raw materials tend to develop pores by carbonization or activation. Furthermore, since the use thereof is a secondary use of waste, they can be procured at low cost. Baked products derived from synthetic resins such as tires, petroleum pitch, urethane resins, phenol resins, and coal can also be used as raw materials.

The activated carbon raw material is carbonized by heating in a temperature range of 200°C to 600 °C, if necessary, to form fine pores. Next, the activated carbon raw material is exposed to steam and carbon dioxide gas in a temperature range of 600°C to 1200 °C and activated. As a result, activated carbon having various developed pores is completed. Alternatively, for example, zinc chloride activation can be used as the activation. Furthermore, sequential cleaning is also performed.

The physical properties of the activated carbon produced in this manner define the adsorption performance of the substance to be adsorbed. The adsorption performance of the activated carbon for adsorbing neutral per- and polyfluoroalkyl compounds, which are the target substances to be adsorbed of the present invention, is defined by the specific surface area, which is an index representing the amount of pores formed in the activated carbon. Note that as used herein, the specific surface area of each preparation example is measured by the BET method (Brunauer, Emmett and Teller method). The higher the adsorption/desorption performance of the activated carbon as a solid phase adsorbent, the more accurate the quantitative analysis of neutral per- and polyfluoroalkyl compounds in a water sample.

The activated carbon is also defined by the pore size of the pores. In the case of an adsorbent material such as activated carbon, all of micropores, mesopores, and macropores are present. Depending on which range of pores among these are better developed, the target and performance of activated carbon adsorb will change. The activated carbon of the present invention is desired to effectively and desorbably adsorb molecules of neutral per- and polyfluoroalkyl compounds.

Furthermore, acidic functional groups are present on the surface of activated carbon. The acidic functional groups, which increase due to surface oxidation of the activated carbon, are primarily hydrophilic groups such as carboxyl groups and phenolic hydroxyl groups. Acidic functional groups on the surface of the activated carbon affect the collection capability. The amount of these acidic functional groups can be understood as the surface oxide amount. When the surface oxide amount of the activated carbon is increased, the hydrophilicity of the surface of activated carbon is increased, and it is considered that the collection performance of fluorotelomer alcohols having a hydrophilic group in particular among neutral per- and polyfluoroalkyl compounds is improved. In particular, by setting the surface oxide amount of the activated carbon as a solid phase adsorbent to 0.10 meq/g or more, neutral per- and polyfluoroalkyl compounds, which are the target substances, can be efficiently adsorbed.

Examples of the method for increasing the surface oxide of activated carbon include the following methods. One is a method of promoting the oxidation of surface residues by repeating the heating step to increase the number of acidic functional groups. This is oxidation under an air or oxygen atmosphere. Alternatively, at the same time, air having a temperature of 25 to 40 °C and a humidity of 60 to 90% is also introduced under an air atmosphere. Then, by heating at 150 to 900 °C for 1 to 10 hours, activated carbon having an increased surface oxide amount can be obtained. It is considered that due to heating with moist air, hydrocarbon groups such as alkyl groups present on the surface of the activated carbon are oxidized and hydroxyl groups of water are introduced to the surface, whereby the amount of acidic functional groups is increased.

Another method is to oxidize the surface of the activated carbon with an oxidizing agent to increase the surface oxides. Examples of the oxidizing agent include hypochlorous acid and hydrogen peroxide. Activated carbon having an increased surface oxide amount can be obtained by immersing activated carbon in a liquid containing such an oxidizing agent and then drying. The amount of acidic functional groups on the surface of the activated carbon can be measured as the surface oxide amount, as shown in each Preparation Example described later.

The adsorption performance of the activated carbon for desorbably adsorbing neutral per- and polyfluoroalkyl compounds is exhibited by setting the specific surface area to 900 $m^2$/g or more. When the pores of the activated carbon are formed to a certain level or more, the adsorption performance of the compounds is secured.

Further, it was found that the distribution of pores formed in the activated carbon also contributes to the adsorption of neutral per- and polyfluoroalkyl compounds. As used herein, "micropores" refers to pores having a pore diameter of 1 nm or less, and as can be derived from the Examples described later, when the total pore volume ($V_{mic}$) of the micropores is 0.35 $cm^3$/g or more, the adsorption performance of neutral per- and polyfluoroalkyl compounds is improved. As used herein, the volume of micropores of 1 nm or less in each Preparation Example is measured by the MP method (Micropore method). It is considered that the compounds are easily collected in the pores when micropores are formed to a certain level or more.

Furthermore, as used herein, "mesopores" refers to pores having a pore diameter in the range of 2 to 60 nm, and as can be derived from the Examples described later, when the total pore volume ($V_{met}$) of the mesopores is 0.02 $cm^3$/g or more, the adsorption performance of neutral per- and polyfluoroalkyl compounds is improved. As used herein, the volume of mesopores in the range of 2 to 60 nm of each Preparation Example is measured by the DH method (Dollimore-Heal method). Since measurement is performed by the DH method, the measurement target is pores of 2.43 to 59.72 nm. It is considered that the compounds can easily penetrate into even the micropores when mesopores are formed to a certain level or more.

Additionally, it is considered that the difference between the pore volume of micropores and the pore volume of mesopores also contributes to the efficient adsorption of neutral per- and polyfluoroalkyl compounds. As derived from the Examples described later, by setting the volume difference ($V_s$) between the sum of the micropore volume ($V_{mic}$) and the sum of the mesopore volume ($V_{met}$) to 0.45 $cm^3$/g or more, the neutral per- and polyfluoroalkyl compounds can be efficiently and desorbably adsorbed. It is considered that by using activated carbon with well-developed micropores in addition to not overdeveloping mesopores, the adsorption performance of neutral per- and polyfluoroalkyl compounds is improved and the compounds can be smoothly desorbed at the time of the subsequent extraction operation, whereby quantitative measurement can be satisfactorily carried out.

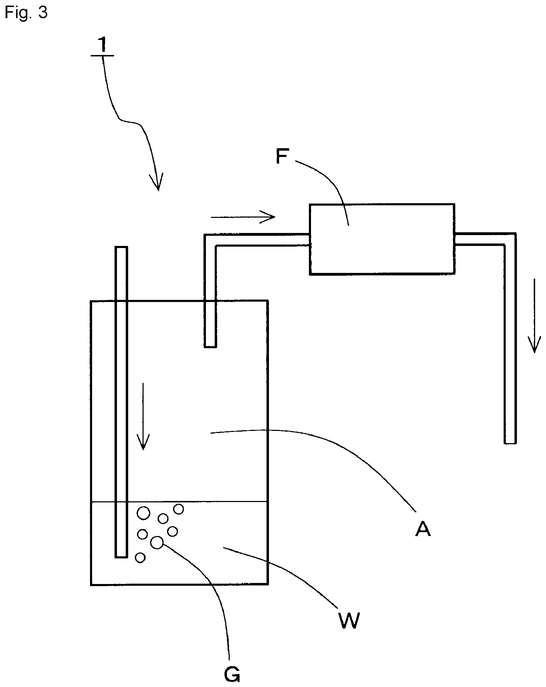

The method for analyzing neutral per- and polyfluoroalkyl compounds in a water sample in the present invention is the purge and trap method. As shown in FIG. 3, in the purge and trap method, as illustrated by the purge and trap device 1, the carrier gas G is passed through the water sample W to distribute the volatile components from the water sample W into the gas phase A, and the adsorbent F arranged downstream adsorbs the volatile components. The gas moves in the direction of the arrow in the drawing. This is a method for measuring and analyzing volatile components in a water sample by desorbing, by heating or with a solvent, volatile components adsorbed by an adsorbent.

In the analysis method of the present invention, a water sample containing neutral per- and polyfluoroalkyl compounds, which are the measurement targets, is aerated or bubbled with an inert gas as a carrier gas, to distribute the neutral per- and polyfluoroalkyl compounds into the gas phase from the water sample. For example, nitrogen gas, argon gas, etc., is used as the inert gas. Aeration with the inert gas may be performed for 30 to 120 minutes at a flow rate of 1 to 10 L/min. If the flow rate is excessively low, the distribution of the neutral per- and polyfluoroalkyl compounds from the water sample into the gas phase is insufficient, and if the flow rate is excessively high, the adsorption of the target substance by the adsorbent arranged downstream may not be able to maintain pace. If the aeration time is excessively short, the neutral per- and polyfluoroalkyl compounds may remain in the water sample, and thus, it is necessary to continue aeration for a certain period of time. Though the aeration time of the inert gas depends on the flow rate and the temperature of the water sample, it is considered that approximately 10 to 60 minutes is sufficient. It is presumed that if the aeration time is excessively long, the amount of volatilized water will increase and the activated carbon will adsorb the water, resulting in poor desorption efficiency during the extraction operation.

Examples of the neutral per- and polyfluoroalkyl compounds present in the water sample include polyfluoroalkyl compounds such as FTOH described above and fluorine telomer iodine (FTI), which is described later, and perfluoroalkyl compounds such as perfluoroalkylsulfonic acid amides (N-MeFOSA and NEtFOSA) and brominated PFASs. Since the distribution of these compounds from the water sample into the gas phase due to the bubbling of the carrier gas may depend on the water temperature, if the distribution thereof into the gas phase is difficult at ambient temperature, the temperature can be appropriately controlled. As shown in the Examples, which are described later, perfluoroalkylsulfonic acid amides are efficiently distributed in the gas phase when the temperature is controlled to approximately 40 °C. Moreover, if the temperature of the water sample is excessively high, water vapor may be generated, which may adversely affect the analysis results. Thus, the water sample can be efficiently analyzed when the temperature is controlled in the range of approximately 25 (ambient temperature) to 60 °C.

The neutral per- and polyfluoroalkyl compounds distributed from the water sample into the gas phase are adsorbed by the solid phase adsorbent arranged downstream. As the solid phase adsorbent, in addition to the activated carbon described above, a polymer-based adsorbent can be used.

Examples of polymer-based adsorbents include styrene-divinylbenzene copolymers, and are mainly reverse-phase solid-phase adsorbents used for separating hydrophobic components from polar solvents. In the same manner as the activated carbon, polymer-based adsorbents have a porous structure and adsorb hydrophobic components due to the action of intermolecular forces (van der Waals forces) as the main interaction.

The neutral per- and polyfluoroalkyl compounds distributed into the gas phase from the water sample adsorbed by the solid phase adsorbent are extracted with an organic solvent and measured. Examples of organic solvents include dichloromethane, ethyl acetate, methanol, and mixed solvents thereof.

EXAMPLES

[Investigation of Neutral Per- and Polyfluoroalkyl Compounds Collection Performance of Activated Carbon]

First, the inventors believed that by enhancing the neutral per- and polyfluoroalkyl compound collection performance of activated carbon as a solid phase adsorbent, the accuracy of quantitative analysis of such compounds can be improved, and performed collection experiments of per- and polyfluoroalkyl compounds in an atmospheric sample.

[Activated Carbon Adsorbents Used]

The inventors used the following raw materials to produce per- and polyfluoroalkyl compound-adsorbing activated carbons.

- Fiber-Like Activated Carbon

   Fiber-like activated carbon "CF" (average fiber diameter: 15 $\mu$m): produced by Futamura Chemical Co., Ltd. {hereinafter referred to as C1};
   Fiber-like activated carbon "FE3010" (average fiber diameter: 15 $\mu$m): produced by Futamura Chemical Co., Ltd. {hereinafter referred to as C2};
   Fiber-like activated carbon "FE3012" (average fiber diameter: 15 $\mu$m): produced by Futamura Chemical Co., Ltd. {hereinafter referred to as C3};
   Fiber-like activated carbon "FE3013" (average fiber diameter: 15 $\mu$m): produced by Futamura Chemical Co., Ltd. {hereinafter referred to as C4};
   Fiber-like activated carbon "FE3015" (average fiber diameter: 15 $\mu$m): produced by Futamura Chemical Co., Ltd. {hereinafter referred to as C5};
   Fiber-like activated carbon "FE3018" (average fiber diameter: 15 $\mu$m): produced by Futamura Chemical Co., Ltd. {hereinafter referred to as C6};

- Granular Activated Carbon

   Coconut shell activated carbon "CW480SZ" (average particle size: 250 $\mu$m): produced by Futamura Chemical Co., Ltd. {hereinafter referred to as C7}; and
   Phenolic resin activated carbon "QW250" (average particle size: 250 $\mu$m): produced by Futamura Chemical Co., Ltd. {hereinafter referred to as C8}.

[Production of Preparation Examples]

<Preparation Example 1>

10 g of the fiber-like activated carbon "CF" (C1) produced by Futamura Chemical was used as the activated carbon of Preparation Example 1.

<Preparation Example 2>

10 g of the fiber-like activated carbon "CF" (C1) produced by Futamura Chemical was immersed in 500 mL of a 4.2% concentration hydrogen peroxide solution, allowed to stand for 220 hours, then removed and dried to obtain the activated carbon of Preparation Example 2.

<Preparation Example 3>

10 g of the fiber-like activated carbon "FE3010" (C2) produced by Futamura Chemical was used as the activated carbon of Preparation Example 3.

<Preparation Example 4>

10 g of the fiber-like activated carbon "FE3010" (C2) produced by Futamura Chemical was immersed in 500 mL of a 4.2% concentration hydrogen peroxide solution, allowed to stand for 150 hours, then removed and dried to obtain the activated carbon of Preparation Example 4.

<Preparation Example 5>

10 g of the fiber-like activated carbon "FE3012" (C3) produced by Futamura Chemical was used as the activated carbon of Preparation Example 5.

<Preparation Example 6>

10 g of the fiber-like activated carbon "FE3012" (C3) produced by Futamura Chemical was immersed in 500 mL of a 4.2% concentration hydrogen peroxide solution, allowed to stand for 100 hours, then removed and dried to obtain the activated carbon of Preparation Example 6.

<Preparation Example 7>

10 g of the fiber-like activated carbon "FE3013" (C4) produced by Futamura Chemical was immersed in 500 mL of a 1.5% concentration hydrogen peroxide solution, allowed to stand for 70 hours, then removed and dried to obtain the activated carbon of Preparation Example 7.

<Preparation Example 8>

10 g of the fiber-like activated carbon "FE3015" (C5) produced by Futamura Chemical was used as the activated carbon of Preparation Example 8.

<Preparation Example 9>

10 g of the fiber-like activated carbon "FE3015" (C5) produced by Futamura Chemical was immersed in 500 mL of a 1.5% concentration hydrogen peroxide solution, allowed to stand for 40 hours, then removed and dried to obtain the activated carbon of Preparation Example 9.

<Preparation Example 10>

10 g of the fiber-like activated carbon "FE3015" (C5) produced by Futamura Chemical was immersed in 500 mL of a 4.2% concentration hydrogen peroxide solution, allowed to stand for 70 hours, then removed and dried to obtain the activated carbon of Preparation Example 10.

<Preparation Example 11>

10 g of the fiber-like activated carbon "FE3015" (C5) produced by Futamura Chemical was immersed in 500 mL of a 14.0% concentration hydrogen peroxide solution, allowed to stand for 350 hours, then removed and dried to obtain the activated carbon of Preparation Example 11.

<Preparation Example 12>

10 g of the fiber-like activated carbon "FE3015" (C5) produced by Futamura Chemical was immersed in 500 mL of a 18.9% concentration hydrogen peroxide solution, allowed to stand for 480 hours, then removed and dried to obtain the activated carbon of Preparation Example 12.

<Preparation Example 13>

10 g of the fiber-like activated carbon "FE3018" (C6) produced by Futamura Chemical was used as the activated carbon of Preparation Example 13.

<Preparation Example 14>

10 g of the fiber-like activated carbon "FE3018" (C6) produced by Futamura Chemical was immersed in 500 mL of a 4.2% concentration hydrogen peroxide solution, allowed to stand for 50 hours, then removed and dried to obtain the activated carbon of Preparation Example 14.

<Preparation Example 15>

10 g of the coconut shell activated carbon "CW480SZ" (C7) produced by Futamura Chemical was immersed in 500 mL of a 4.2% concentration hydrogen peroxide solution, allowed to stand for 70 hours, then removed and dried to obtain the activated carbon of Preparation Example 15.

<Preparation Example 16>

10 g of the phenolic resin activated carbon "QW250" (C8) produced by Futamura Chemical was immersed in 500 mL of a 4.2% concentration hydrogen peroxide solution, allowed to stand for 70 hours, then removed and dried to obtain the activated carbon of Preparation Example 16.

[Surface Oxide Amount]

For the surface oxide amount (meq/g), using the Boehm method, the adsorbing activated carbon of each Example was shaken in a 0.05 N sodium hydroxide aqueous solution, filtered, and the filtrate was neutralized with 0.05 N hydrochloric acid. The amount of sodium hydroxide at the time of titration was used as the surface oxide amount.

[BET Specific Surface Area]

The specific surface area ($m^2/g$) was determined by the BET method (BET specific surface area) by measuring the nitrogen adsorb isotherm at 77 K using the automatic specific surface area/pore distribution measuring device "BELSORP-mini II" produced by MicrotracBEL Corporation.

[Average Pore Diameter]

The average pore diameter (nm) was calculated from formula (iv) using the values of the pore volume ($cm^3/g$) and the specific surface area ($m^2/g$), assuming that the shape of the pores is cylindrical.
[Formula 4]

$$\text{Average pore diameter (nm)} = \left( \frac{\text{Pore volume } (cm^3/g)}{\text{Specific Surface Area } (m^2/g)} \right) \times 4 \times 1000 \quad \cdots \quad (iv)$$

[Micropore Volume]

The pore volume was measured by nitrogen adsorption using an automatic specific surface area/pore distribution measuring device ("BELSORP-mini II", manufactured by MicrotracBEL Corporation). The sum of the micropore volume ($V_{mic}$) ($cm^3/g$), which is the pore volume in the range of pore diameters of 1 nm or less, of Preparation Examples 1 to 16, was obtained by analyzing the value of dV/dD in the range of pore diameters of 1 nm or less from the t-plot of the adsorption isotherm of nitrogen gas by the MP method.

[Mesopore Volume]

The dV/dD values in the pore diameter range of 2 to 60 nm were analyzed by the DH method from the adsorption isotherm of nitrogen gas. The diameter range of the pore diameters of 2 to 60 nm in the analysis software is 2.43 to 59.72 nm. From this analysis result, the sum ($V_{met}$) ($cm^3$/g) of the mesopore volume, which is the pore volume in the range of pore diameters of 2 to 60 nm, of each of Preparation Examples 1 to 16 was obtained.

[Volume Difference]

The volume difference ($V_s$) of each of Preparation Examples 1 to 16 is a value obtained by subtracting the sum of the mesopore volume ($V_{met}$) ($cm^3$/g) from the sum of the micropore volume ($V_{mic}$) ($cm^3$/g), and was calculated from the above formula (i).

Tables 1 to 3 show the physical characteristics of the activated carbons of Preparation Examples 1 to 16. In Tables 1 to 3, from the top, the surface oxide amount (meq/g), BET specific surface area ($m^2$/g), average pore diameter (nm), micropore volume ($V_{mic}$) ($cm^3$/g), mesopore volume ($V_{met}$) ($cm^3$/g), and volume difference ($V_s$) ($cm^3$/g) are shown.

[Table 1]

| | | Prep Ex 1 | Prep Ex 2 | Prep Ex 3 | Prep Ex 4 | Prep Ex 5 | Prep Ex 6 |
|---|---|---|---|---|---|---|---|
| Surface oxidation amount | (meq/g) | 0.05 | 0.30 | 0.10 | 0.41 | 0.15 | 0.57 |
| BET specific surface area | ($m^2$/g) | 526 | 482 | 825 | 769 | 951 | 989 |
| Average pore diameter | (nm) | 1.94 | 1.98 | 1.54 | 1.53 | 1.59 | 1.61 |
| Micropore volume ($V_{mic}$) | ($cm^3$/g) | 0.195 | 0.168 | 0.345 | 0.315 | 0.380 | 0.394 |
| Mesopore volume ($V_{met}$) | ($cm^3$/g) | 0.069 | 0.074 | 0.001 | 0.004 | 0.026 | 0.031 |
| Volume difference ($V_s$) | ($cm^3$/g) | 0.126 | 0.094 | 0.344 | 0.311 | 0.354 | 0.363 |

[Table 2]

| | | Prep Ex 7 | Prep Ex 8 | Prep Ex 9 | Prep Ex 10 | Prep Ex 11 |
|---|---|---|---|---|---|---|
| Surface oxidation amount | (meq/g) | 0.19 | 0.10 | 0.20 | 0.48 | 0.92 |
| BET specific surface area | ($m^2$/g) | 1320 | 1572 | 1543 | 1474 | 1356 |
| Average pore diameter | (nm) | 1.65 | 1.76 | 1.65 | 1.64 | 1.73 |
| Micropore volume ($V_{mic}$) | ($cm^3$/g) | 0.531 | 0.796 | 0.641 | 0.612 | 0.546 |
| Mesopore volume ($V_{met}$) | ($cm^3$/g) | 0.048 | 0.094 | 0.038 | 0.041 | 0.036 |
| Volume difference ($V_s$) | ($cm^3$/g) | 0.483 | 0.702 | 0.603 | 0.571 | 0.510 |

[Table 3]

| | | Prep Ex 12 | Prep Ex 13 | Prep Ex 14 | Prep Ex 15 | Prep Ex 16 |
|---|---|---|---|---|---|---|
| Surface oxidation amount | (meq/g) | 1.10 | 0.10 | 0.42 | 0.38 | 0.86 |
| BET specific surface area | ($m^2$/g) | 1341 | 2017 | 1949 | 1700 | 1966 |
| Average pore diameter | (nm) | 1.71 | 1.89 | 1.84 | 1.99 | 2.08 |
| Micropore volume ($V_{mic}$) | ($cm^3$/g) | 0.545 | 0.841 | 0.838 | 0.679 | 0.729 |
| Mesopore volume ($V_{met}$) | ($cm^3$/g) | 0.075 | 0.124 | 0.117 | 0.234 | 0.414 |
| Volume difference ($V_s$) | ($cm^3$/g) | 0.470 | 0.717 | 0.721 | 0.445 | 0.315 |

[Measurement of Neutral Per- and Polyfluoroalkyl Compound Collection Efficiency in Atmospheric Sample]

Evaluation of Preparation Examples 1 to 16 was performed using, for this evaluation, fluorotelomer alcohols (hereinafter referred to as "FTOHs") as the neutral per- and polyfluoroalkyl compounds. FTOHs are substances represented by the above-mentioned chemical formula (ii), and the substance names differ depending on the number of carbon atoms. For example, in the case of $C_8F_{17}CH_2CH_2OH$, this FTOH is referred to as 8:2 FTOH (IUPAC name: 3,3,4,4,5,5,6,6,7,7,8,8,9,9,10,10,10-heptadecafluoro-1-decanol).

Each standard substance was diluted with methanol to 100 ng/mL (100 ppb), 100 μL thereof was added to flexible polyurethane foam (PUF), and this was set in the first stage. Next, the activated carbon of the Preparation Example was filled in a case having a diameter of 47 mm in the second stage so that the thickness at the time of filling became approximately 2 mm, and the first stage PUF and the second stage fiber-like activated carbon were aerated with air at 22 to 24 °C at a rate of 20 L/min for 48 hours.

After aeration, the activated carbon of the Preparation Example was transferred to a PP centrifuge tube (capacity: 15 mL), and 10 mL of a mixed solvent containing dichloromethane and ethyl acetate as main components was added thereto. The centrifuge tube was shaken at 225 rpm for 10 minutes, and an extract was then collected. This extraction process was repeated twice in succession, and a total of 30 mL of extract was collected.

The collected extract was concentrated to 1 mL by a nitrogen blowing concentrator, and the extract was then quantitatively measured using a GC-MS/MS ("GCMS-TQ8050", manufactured by Shimadzu Corporation) in MRM mode, and the collection performance was confirmed.

Tables 4 to 6 show the recovery rate (%) of fluorotelomer alcohols (FTOHs) for each target substance for the activated carbons of Preparation Examples 1 to 16. The target substances are 4:2 FTOH (IUPAC name: 3,3,4,4,5,5,6,6,6-nonafluoro-1-hexanol), 4:3 FTOH (IUPAC name: 4,4,5,5,6,6,7,7,7-nonanofluoro-1-heptanol), 6:2 FTOH (IUPAC name: 3,3,4,4,5,5,6,6,7,7,8,8,8-tridecafluoro-1-octanol), 6:3 FTOH (IUPAC name: 4,4,5,5,6,6,7,7,8,8,9,9,9-tridecafluoro-1-nonanol), 8:2 FTOH (IUPAC name: 3,3,4,4,5,5,6,6,7,7,8,8,9,9,10,10, 10-heptadecafluoro-1-decanol), 8:3 (IUPAC name: 4,4,5,5,6,6,7,7,8,8,9,9,10,10,11,11,11-heptadecafluoro-1-undecanol), and 10:2 FTOH (IUPAC name: 3,3,4,4,5,5,6,6,7,7,8,8,9,9,10,10,11,11,12,12,12-heneicosafluoro-1-dodecanol). Note that in the tables, "ND" indicates that the value is below the quantifiable lower limit.

[Table 4]

| | | Prep Ex 1 | Prep Ex 2 | Prep Ex 3 | Prep Ex 4 | Prep Ex 5 | Prep Ex 6 |
|---|---|---|---|---|---|---|---|
| Target Substance | 4:2 FTOH | ND | ND | ND | ND | 56% | 65% |
| | 6:2 FTOH | ND | ND | ND | ND | 60% | 64% |
| | 8:2 FTOH | ND | ND | ND | ND | 77% | 71% |
| | 10:2 FTOH | ND | ND | ND | ND | 61% | 53% |

[Table 5]

| | | Prep Ex 7 | Prep Ex 8 | Prep Ex 9 | Prep Ex 10 | Prep Ex 11 |
|---|---|---|---|---|---|---|
| Target Substance | 4:2 FTOH | 83% | 66% | 79% | 79% | 74% |
| | 6:2 FTOH | 73% | 77% | 78% | 87% | 70% |
| | 8:2 FTOH | 98% | 58% | 78% | 87% | 81% |
| | 10:2 FTOH | 90% | 92% | 83% | 93% | 79% |

[Table 6]

| | | Prep Ex 12 | Prep Ex 13 | Prep Ex 14 | Prep Ex 15 | Prep Ex 16 |
|---|---|---|---|---|---|---|
| Target Substance | 4:2 FTOH | 71% | 75% | 79% | 45% | 45% |
| | 6:2 FTOH | 98% | 57% | 74% | 62% | 62% |
| | 8:2 FTOH | 89% | 74% | 87% | 51% | 51% |
| | 10:2 FTOH | 89% | 85% | 85% | 39% | 39% |

[Results and Discussion]

In Preparation Examples 1 to 4, the recovery rate was below the lower limit of quantification for each of the FTOHs, and the adsorption of the target substances was insufficient. It is presumed that the adsorption performance was not exhibited due to a lack of pores or specific surface area required for the adsorption of the target substances.

Preparation Examples 5 to 16 were capable of recovering each of FTOHs. When the BET specific surface area was 900 $m^2/g$ or more, it was shown that the target substances were adsorbed. It is inferred that the parameter of the specific surface area of activated carbon has a certain effect on the adsorption performance of each FTOH. In particular, Preparation Examples 5 to 14, which were fiber-like activated carbons, showed good results of 50% or more in the recovery rate of FTOHs. From the viewpoint of the contact efficiency between the target substance and the activated carbon, it is considered that FTOH adsorption can be performed more efficiently by using fiber-like activated carbon.

It was also shown that the adsorption performance of FTOHs was higher when activated carbon with developed micropores and mesopores was used. In Preparation Examples 1 and 2, it is considered that since neither micropores nor mesopores were developed, no FTOH was adsorbed. In Preparation Examples 3 and 4, it is considered that since micropores were developed but mesopores were not, the number of mesopores present on the inlet side of the pores of the activated carbon was small, whereby the molecules of the FTOHs were not smoothly introduced into the micropores and were not adsorbed.

Since it is considered that Preparation Examples 5 to 16 had large pore volumes of both micropores and mesopores and both pores were sufficiently developed, it can be inferred that the molecules of the FTOHs were smoothly introduced into the pores of the activated carbon and excellent adsorption performance was demonstrated. Preparation Examples 7 to 14 demonstrated particularly excellent performance in the recovery of FTOHs. Preparation Examples 7 to 14 are each characterized in that the pore volume of the micropores is large and the pore volume of the mesopores is not particularly large though the pores of the mesopores are developed. It is considered that after adsorption of the molecules of the FTOHs in the micropores, the molecules were likely to be smoothly desorbed out of the pores during the extraction operation, and thus, a particularly good recovery rate was demonstrated.

Conversely, Preparation Examples 15 and 16 are considered to be activated carbons having pores developed in a complicated manner from large pores to small pores because the pore volumes of both the micropores and the mesopores are large. It is presumed that the molecules of the FTOHs adsorbed in the complicatedly developed pores were less likely to be smoothly desorbed during the extraction operation, and the rates of recovery of the FTOHs were slightly inferior to those of Preparation Examples 7 to 14. In light of these results, it can be understood that the sum of the pore volumes of the micropores ($V_{mic}$) of the activated carbon, the sum of the pore volume of the mesopores ($V_{met}$), and the volume difference (Vs), which is the difference therebetween, affect the FTOH recovery rate.

In addition to the pore conditions of the activated carbon, it was investigated whether improving the surface oxide amount would improve affinity with FTOHs having a hydrophilic group and improve the FTOH adsorption performance. Regarding Preparation Example 8 and Preparation Examples 9 to 12, which had the same activated carbon raw material, Preparation Examples 9 to 12, in which the surface oxide amount was increased, demonstrated better adsorption performance. Likewise, regarding Preparation Example 13 and Preparation Example 14, Preparation Example 14 having a large surface oxide amount demonstrated better adsorption performance. Thus, it can be understood that it is possible to further improve the FTOH adsorption performance by increasing the surface oxide amount of the activated carbon.

[Quantitative Measurement of Neutral Per- and Polyfluoroalkyl Compounds in Water Sample]

Next, the inventors conducted analytical experiments aimed at quantitative measurement of neutral per- and polyfluoroalkyl compounds in a water sample by the purge and trap method using the following solid phase adsorbent.

[Solid Phase Adsorbent]

The inventors used the following activated carbon and polymer-based adsorbents as solid phase adsorbents for adsorbing neutral per- and polyfluoroalkyl compounds.

<Activated Carbon>

10 g of the fiber-like activated carbon "FE3015" (average fiber diameter: 15 μm) produced by Futamura Chemical was immersed in 500 mL of a 4.2% concentration hydrogen peroxide solution, allowed to stand for 70 hours, then removed and dried to obtain an activated carbon. 90 mg of this activated carbon was filled in a disk-shaped solid phase column. The physical properties of the activated carbon are as described below. Each physical property is determined as described above.

Surface oxide amount: 0.48 (meq/g)
BET specific surface area: 1474 ($m^2/g$)
Average pore diameter: 1.64 (nm)

<Polymer-Based Adsorbent>

An "InertSep® Slim-JPLS-2", manufactured by GL Sciences Inc., in which a styrene-divinylbenzene copolymer is filled, was used.

[Analysis of Neutral Per- and Polyfluoroalkyl Compounds in Water Sample]

Evaluation was performed using, for this evaluation, FTOHs, ethylperfluorooctane sulfonamide (IUPAC name: N-ethyl-1,1,2,2,3,3,4,4,5,5,6,6,7,7,8,8,8-heptadecafluoro octane-1-sulfonamide) (hereinafter referred to as "N-EtFOSA"), methylperfluorooctanesulfonamide (IUPAC name: 1,1,2,2,3,3,4,4,5,5,6,6,7,7,8,8,8-heptadecafluoro-N-methyl-1-octanesulfonamide (hereinafter referred to as "N-MeFOSA"), fluorine telomer iodines (hereinafter referred to as "FTIs"), and brominated PFASs. N-EtFOSA is a substance represented by the following chemical formula (v). N-MeFOSA is a substance represented by the following chemical formula (vi). FTI is a substance represented by the following chemical formula (vii).

[Formula 5]

$$C_8F_{17} - SO_2 - N(C_2H_5) - H \cdots \qquad \text{(v)}$$

[Formula 6]

$$C_8F_{17} - SO_2 - N(CH_3) - H \cdots \qquad \text{(vi)}$$

[Formula 7]

$$C_nF_{2n+1} - C_2H_4 - I \cdots \qquad \text{(vii)}$$

Each standard substance was diluted with methanol to 100 ng/mL (100 ppb), and 100 $\mu$L thereof was added to 300 mL of ultrapure water to produce a water sample of 33 ng/L (33 ppt). The produced water sample was sealed in a 1 L purge vessel, and a carrier gas supply line was set. Nitrogen gas was used as the carrier gas. The water temperature, gas flow rate, and aeration time were appropriately controlled, and each standard substance distributed in the gas phase was collected by aerating the solid phase column filled with the solid phase adsorbent and arranged downstream. After aeration, 14 mL of a mixed solvent containing dichloromethane and ethyl acetate as main components was passed through each solid phase column at a rate of 1 drop/second, and the extract was collected.

The collected extract was concentrated to 1 mL by a nitrogen blowing concentrator, and the extract was then quantitatively measured using a GC-MS/MS ("GCMS-TQ8050", manufactured by Shimadzu Corporation) in MRM mode, and the collection performance was confirmed.

The inventors conducted analytical experiments aimed to measure the recovery rate of the neutral per- and polyfluoroalkyl compounds in the water samples under the conditions described in Test Examples 1 to 9 below.

<Test Example 1>

The analysis experiment of Test Example 1 was conducted by aerating the water sample with the carrier gas at a flow rate of 2 L/min for 30 minutes using activated carbon as the solid phase adsorbent, at a water temperature of the water sample of 25 °C (ambient temperature).

<Test Example 2>

The analysis experiment of Test Example 2 was conducted by aerating the water sample with the carrier gas at a flow rate of 2 L/min for 30 minutes using activated carbon as the solid phase adsorbent, while controlling the water temperature of the water sample at 40 °C (ambient temperature).

<Test Example 3>

The analysis experiment of Test Example 3 was conducted by aerating the water sample with the carrier gas at a flow rate of 2 L/min for 30 minutes using activated carbon as the solid phase adsorbent, while controlling the water temperature of the water sample at 10 °C.

<Test Example 4>

The analysis experiment of Test Example 4 was conducted by aerating the water sample with the carrier gas at a flow rate of 2 L/min for 60 minutes using activated carbon as the solid phase adsorbent, while controlling the water temperature of the water sample at 40 °C.

<Test Example 5>

The analysis experiment of Test Example 5 was conducted by aerating the water sample with the carrier gas at a flow rate of 2 L/min for 120 minutes using activated carbon as the solid phase adsorbent, while controlling the water temperature of the water sample at 40 °C.

<Test Example 6>

The analysis experiment of Test Example 6 was conducted by aerating the water sample with the carrier gas at a flow rate of 1 L/min for 30 minutes using activated carbon as the solid phase adsorbent, while controlling the water temperature of the water sample at 40 °C.

<Test Example 7>

The analysis experiment of Test Example 7 was conducted by aerating the water sample with the carrier gas at a flow rate of 4 L/min for 30 minutes using activated carbon as the solid phase adsorbent, while controlling the water temperature of the water sample at 40 °C.

<Test Example 8>

The analysis experiment of Test Example 8 was conducted by aerating the water sample with the carrier gas at a flow rate of 8 L/min for 30 minutes using activated carbon as the solid phase adsorbent, while controlling the water temperature of the water sample at 40 °C.

<Test Example 9>

The analysis experiment of Test Example 9 was conducted by aerating the water sample with the carrier gas at a flow rate of 0.8 L/min for 30 minutes using a polymer-based adsorbent as the solid phase adsorbent, while controlling the water temperature of the water sample at 40 °C.

<Comparative Example 1>

Furthermore, as Comparative Example 1, an experiment was conducted by the solid-phase extraction method. 300 mg of the fiber-like activated carbon of Preparation Example 10 was filled in a 6 mL syringe (13 mm in diameter) to form a solid-phase extraction column. Each standard substance was diluted with methanol to 100 ng/mL (100 ppb), and 100 $\mu$L of the standard solution was added to 100 mL of ultrapure water to produce a water sample of 100 ng/L (100 ppt) in a PP bottle.

As pre-cleaning of the solid phase adsorbent, 15 mL of a mixed solvent containing dichloromethane and ethyl acetate as main components was passed at a rate of 1 drop/second, and subsequently, 5 mL of methanol and 15 mL of ultrapure water were passed in this order in the same manner.

Next, 100 mL of the produced water sample was passed through the solid phase adsorbent at a rate of 1 drop/second, and thereafter, 5 mL of ultrapure water was added to the PP bottle in which the water sample was produced, and the target substance remaining in the PP bottle was washed away by passing water through the bottle again. After passing the liquid therethrough, water in the solid phase column was removed by centrifugation (3500 rpm, 30 minutes).

After centrifugation, 4 mL of the methanol solution was added into the PP bottle to wash out the target substance remaining on the inner wall, and the solution was passed through the solid phase column after dehydration at a rate of 1 drop/second to collect an extract 1. Subsequently, 15 mL of a mixed solvent containing dichloromethane and ethyl acetate as main components was passed in the same manner to obtain an extract 2. The collected extracts were concentrated to 1 mL with a nitrogen blowing concentrator. After concentration, 50 mg of mirabilite ($Na_2SO_4$) was added to the extract 1 to remove contaminating water. The extract was then quantitatively measured using a GC-MS/MS ("GCMS-TQ8050", manufactured by Shimadzu Corporation) in MRM mode, and the analysis results were confirmed.

The test conditions of Test Examples 1 to 9 are summarized in Tables 7 and 8.

[Table 7]

|  | Prep Ex 1 | Prep Ex 2 | Prep Ex 3 | Prep Ex 4 | Prep Ex 5 |
|---|---|---|---|---|---|
| Solid phase adsorbent type | Activated carbon | Activated carbon | Activated carbon | Activated carbon | Activated carbon |
| Water temp (°C) | 25 | 40 | 10 | 40 | 40 |
| Flow rate (L/min) | 2 | 2 | 2 | 2 | 2 |
| Aeration time (min) | 30 | 30 | 30 | 60 | 120 |

[Table 8]

|  | Prep Ex 6 | Prep Ex 7 | Prep Ex 8 | Prep Ex 9 |
|---|---|---|---|---|
| Solid phase adsorbent type | Activated carbon | Activated carbon | Activated carbon | Polymer-based adsorbent |
| Water temp (°C) | 40 | 40 | 40 | 40 |
| Flow rate (L/min) | 1 | 4 | 8 | 0.8 |
| Aeration time (min) | 30 | 30 | 30 | 30 |

The recovery rates (%) of Test Examples 1 to 9 and Comparative Example 1 for each target substance are shown in Tables 9 and 10. The target substances were 4:2 FTOH, 4:3 FTOH, 6:2 FTOH, 6:3 FTOH, 8:2 FTOH, 8:3 FTOH, 10:2 FTOH, N-EtFOSA, N-MeFOSA, 6:2 FTI, 8:2 FTI, 10:2 FTI, $C_8R_3BrF_6$, and $C_6BrF_5$. Note that "-" in the Tables indicates that the experiment was not conducted.

[Table 9]

|  |  | Prep Ex 1 | Prep Ex 2 | Prep Ex 3 | Prep Ex 4 | Prep Ex 5 |
|---|---|---|---|---|---|---|
| Target Substance | 4:2 FTOH | 74% | 76% | 100% | 81% | 53% |
|  | 4:3 FTOH | 72% | 75% | 82% | 49% | 32% |
|  | 6:2 FTOH | 76% | 101% | 104% | 115% | 79% |
|  | 6:3 FTOH | 67% | 105% | 114% | 87% | 65% |
|  | 8:2 FTOH | 93% | 108% | 112% | 108% | 74% |
|  | 8:3 FTOH | 84% | 106% | 77% | 102% | 77% |
|  | 10:2 FTOH | 84% | 98% | 57% | 92% | 77% |
|  | N-EtFOSA | 20% | 72% | - | - | - |
|  | N-MeFOSA | 18% | 68% | - | - | - |
|  | 6:2 FTI | 76% | 80% | 72% | 72% | 54% |
|  | 8:2 FTI | 78% | 81% | 75% | 75% | 58% |
|  | 10:2 FTI | 76% | 81% | 62% | 62% | 56% |
|  | $C_8H_3BrF_6$ | 67% | 83% | 75% | 75% | 57% |
|  | $C_6B_rF_5$ | 63% | 74% | 71% | 71% | 73% |

[Table 10]

| Target Substance | | Prep Ex 6 | Prep Ex 7 | Prep Ex 8 | Prep Ex 9 | Comp Ex 1 |
|---|---|---|---|---|---|---|
| | 4:2 FTOH | 59% | 66% | 55% | 68% | 25% |
| | 4:3 FTOH | 29% | 55% | 54% | 62% | 56% |
| | 6:2 FTOH | 84% | 95% | 79% | 71% | 35% |
| | 6:3 FTOH | 54% | 79% | 63% | 57% | 49% |
| | 8:2 FTOH | 56% | 75% | 82% | 64% | 31% |
| | 8:3 FTOH | 59% | 79% | 80% | 57% | 36% |
| | 10:2 FTOH | 58% | 78% | 73% | 55% | 32% |
| | N-EtFOSA | - | - | - | - | 43% |
| | N-MeFOSA | - | - | - | - | 26% |
| | 6:2 FTI | 68% | 72% | 62% | 83% | 22% |
| | 8:2 FTI | 64% | 74% | 62% | 68% | 29% |
| | 10:2 FTI | 62% | 66% | 53% | 52% | 37% |
| | $C_8H_3BrF_6$ | 62% | 70% | 64% | 73% | 24% |
| | $C_6B_rF_5$ | 53% | 63% | 67% | 64% | 0% |

[Results and Discussion]

In each of Test Examples 1 to 9, in which the purge and trap method was used, the recovery rate of neutral per- and polyfluoroalkyl compounds in the water sample was higher than in Comparative Example 1, in which the solid-phase extraction method was used. This indicates that the purge and trap method is suitable as an analysis method for quantitative measurement of neutral per- and polyfluoroalkyl compounds present in a water sample.

Test Examples 1 to 3, in which the temperature conditions were changed, will be compared. In Test Example 3, in which the temperature of the water sample was 10 °C, which is below ambient temperature, and Test Examples 1 and 2, in which the temperature of the water sample was above ambient temperature, the recovery rates of per- and polyfluoroalkyl compounds of FTOHs, FTIs, and brominated PFASs were the same or slightly increased. From this, it can be understood that the quantitative measurement of per- and polyfluoroalkyl compounds does not particularly require temperature control of the water sample. Furthermore, in Test Example 1, which was performed at ambient temperature (25 °C), and Test Example 2, which was performed at 40 °C, the recovery rates of the perfluoroalkyl compounds of N-EtFOSA and N-MeFOSA were significantly improved. Thus, it is desirable that the water sample be controlled to a temperature at which the substance can be distributed in the gas phase according to the target substance to be quantitatively measured. It can be understood that stable quantitative analysis of each compound is possible by controlling the temperature of the water sample to 40 °C or higher.

Next, when Test Examples 2, 4, and 5, in which the inert gas aeration time was changed, are compared with each other, the recovery rate of Test Example 4, in which aeration was performed for 60 minutes, was slightly increased as compared to Test Example 2, in which aeration was performed for 30 minutes. Test Example 5, in which aeration was performed for 120 minutes, exhibited a constant recovery rate, but the recovery rate was reduced as compared to Test Examples 2 and 4. From this, it can be understood that the aeration time should be approximately 10 to 100 minutes, desirably approximately 30 to 60 minutes. It is presumed that the reason why the recovery rate decreased after aeration for 120 minutes was that the amount of volatilized water increased and the water was adsorbed to the activated carbon, resulting in a deterioration in the desorption efficiency during the extraction operation.

When test Examples 2 and 6 to 8, in which the flow rate of the inert gas was changed, are compared with each other, the results revealed that Test Example 6, in which the flow rate was 1 L/min, exhibited a certain recovery rate, but was inferior in recovery rate to test Examples 2, 7, and 8. Further, the results revealed that Test Examples 7 and 8, in which the flow rate was high, were inferior (though slightly) in recovery rate to Test Example 2. It is considered that if the flow rate of the inert gas is excessively low, the distribution of neutral per- and polyfluoroalkyl compounds from the water sample into the gas phase is insufficient, and if the flow rate is excessively high, the adsorption of the compounds by the solid phase adsorbent cannot maintain pace. From this, it can be understood that the flow rate of the inert gas is desirably approximately 2 to 8 L/min, particularly approximately 2 L/min, though it appears to depend on the filling amount of the solid phase adsorbent.

In Preparation Example 9, in which a polymer-based adsorbent was used as the solid phase adsorbent, though a certain recovery rate was exhibited, the pressure loss was high and the inert gas could not be aerated at a flow rate greater than 0.8 L/min. However, since the inert gas flow rate cannot be increased, the recovery rate can only be improved by extending the aeration time, and thus, it was understood that activated carbon is superior as a solid phase adsorbent.

As illustrated above, it was shown that by using the purge and trap method of aerating or bubbling, with an inert gas as a carrier gas, a water sample which is sealed in a vessel and temperature-controlled to a temperature at which neutral per- and polyfluoroalkyl compounds can be distributed in a gas phase to desorbably adsorb neutral per- and polyfluoroalkyl compounds distributed in the gas phase from the water sample, quantitative measurement of the neutral per-and polyfluoroalkyl compounds in the water sample is possible. Further, it was shown that by using an activated carbon having a high perfluoroalkyl compound adsorption performance as the solid phase adsorbent, quantitative analysis of the neutral per- and polyfluoroalkyl compounds in the water sample with higher accuracy is possible.

In particular, when the water sample is temperature-controlled to approximately 25 to 40 °C and inert gas is passed at a flow rate of 2 to 8 L/min for 30 to 60 minutes, a suitable recovery rate of neutral per- and polyfluoroalkyl compounds is exhibited, whereby quantitative analysis of the neutral per- and polyfluoroalkyl compounds in the water sample with higher accuracy is possible.

INDUSTRIAL APPLICABILITY

By using the activated carbon for adsorbing neutral per- and polyfluoroalkyl compounds of the present invention as a solid phase adsorbent in the purge and trap method, it is possible to quantitatively measure volatile neutral per- and polyfluoroalkyl compounds present in a water sample, which is currently considered difficult. Furthermore, by using the activated carbon, which is capable of effectively detachably-adsorbing per- and polyfluoroalkyl compounds, it is possible to perform quantitative measurement of such compounds with further improved accuracy.

DESCRIPTION OF REFERENCE SIGNS

1       purge and trap device
10     disk-type solid phase column
11     intake opening
12     body
20     activated carbon filter part
21     felt-like filter part
A       gas phase
G       carrier gas
W       water sample

**Claims**

1.   An activated carbon for adsorbing neutral per- and polyfluoroalkyl compounds used in quantitative analysis of neutral per- and polyfluoroalkyl compounds in a water sample, comprising an activated carbon having a BET specific surface area of 900 $m^2$/g or more for desorbably adsorbing neutral per- and polyfluoroalkyl compounds distributed in a gas phase from the water sample by aerating or bubbling, with an inert gas as a carrier gas, the water sample sealed in a vessel and temperature-controlled to a temperature at which the neutral per- and polyfluoroalkyl compounds can be distributed in a gas phase.

2.   The activated carbon for adsorbing neutral per- and polyfluoroalkyl compounds according to claim 1, wherein a sum ($V_{mic}$) of a volume of micropores of 1 nm or less of the activated carbon is 0.35 $cm^3$/g or more.

3.   The activated carbon for adsorbing neutral per- and polyfluoroalkyl compounds according to claim 1 or 2, wherein a sum ($V_{met}$) of a volume of mesopores of 2 to 60 nm or less of the activated carbon is 0.02 $cm^3$/g or more.

4.   The activated carbon for adsorbing neutral per- and polyfluoroalkyl compounds according to any one of claims 1 to 3, wherein a volume difference ($V_s$) between the sum ($V_{mic}$) of the volume of micropores and the sum ($V_{met}$) of the volume of mesopores of the activated carbon as prescribed by the following formula (i) is 0.45 $cm^3$/g or more.

[Formula 1]

$$V_s = V_{mic} - V_{met} \quad \cdots (i)$$

5. The activated carbon for adsorbing neutral per- and polyfluoroalkyl compounds according to any one of claims 1 to 4, wherein a surface oxide amount of the activated carbon is 0.10 meq/g or more.

6. A filter body for adsorbing neutral per-and polyfluoroalkyl compounds, comprising a disk-shaped solid phase column filled with the activated carbon for adsorbing neutral per- and polyfluoroalkyl compounds in a water sample according to any one of claims 1 to 5.

7. An analysis method for quantitative analysis of neutral per- and polyfluoroalkyl compounds in a water sample, the method comprising:

aerating or bubbling, with an inert gas as a carrier gas, a water sample sealed in a vessel and temperature-controlled to a temperature at which neutral per- and polyfluoroalkyl compounds can be distributed in a gas phase, distributing the neutral per- and polyfluoroalkyl compounds in the gas phase from the water sample, and adsorbing the neutral per- and polyfluoroalkyl compounds with a solid phase adsorbent, and
subjecting the neutral per- and polyfluoroalkyl compounds adsorbed by the solid phase adsorbent to extraction with an organic solvent and measurement.

8. The analysis method for neutral per- and polyfluoroalkyl compounds in a water sample according to claim 7, wherein the solid phase adsorbent is an activated carbon adsorbent.

9. The analysis method for neutral per- and polyfluoroalkyl compounds in a water sample according to claim 7 or 8, wherein the water sample is temperature-controlled to 25 to 60 °C.

10. The analysis method for neutral per- and polyfluoroalkyl compounds in a water sample according to any one of claims 7 to 9, wherein aerating or bubbling of the inert gas is performed for 10 to 60 minutes.

11. The analysis method for neutral per- and polyfluoroalkyl compounds in a water sample according to any one of claims 7 to 10, wherein a main component of the organic solvent is a mixed solvent of dichloromethane and ethyl acetate.

12. The analysis method for neutral per- and polyfluoroalkyl compounds in a water sample according to any one of claims 8 to 11, wherein the BET specific surface area of the activated carbon is 900 m2/g or more.

13. The analysis method for neutral per- and polyfluoroalkyl compounds in a water sample according to any one of claims 8 to 12, wherein a sum ($V_{mic}$) of a volume of micropores of 1 nm or less of the activated carbon is 0.35 cm$^3$/g or more.

14. The analysis method for neutral per- and polyfluoroalkyl compounds in a water sample according to any one of claims 8 to 13, wherein a sum ($V_{met}$) of a volume of mesopores of 2 to 60 nm or less of the activated carbon is 0.02 cm$^3$/g or more.

15. The analysis method for neutral per- and polyfluoroalkyl compounds in a water sample according to any one of claims 8 to 14, wherein a volume difference ($V_s$) between the sum ($V_{mic}$) of the volume of micropores and the sum ($V_{met}$) of the volume of mesopores of the activated carbon as prescribed by the following formula (i) is 0.45 cm$^3$/g or more.

16. The analysis method for neutral per- and polyfluoroalkyl compounds in a water sample according to any one of claims 8 to 15, wherein a surface oxide amount of the activated carbon is 0.10 meq/g or more.

Fig. 1

Fig. 2

Fig. 3

| | | |
|---|---|---|
| **INTERNATIONAL SEARCH REPORT** | International application No. | |
| | **PCT/JP2021/035076** | |

**A.   CLASSIFICATION OF SUBJECT MATTER**

*C01B 32/306*(2017.01)i; *B01J 20/20*(2006.01)i; *B01J 20/28*(2006.01)i; *G01N 30/88*(2006.01)i
FI:   G01N30/88 X; B01J20/20 B; B01J20/28 Z; C01B32/306

According to International Patent Classification (IPC) or to both national classification and IPC

**B.   FIELDS SEARCHED**

Minimum documentation searched (classification system followed by classification symbols)

C01B32/306; B01J20/20; B01J20/28; G01N30/88

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII)

**C.   DOCUMENTS CONSIDERED TO BE RELEVANT**

| Category* | Citation of document, with indication, where appropriate, of the relevant passages | Relevant to claim No. |
|---|---|---|
| Y | JP 2013-220413 A (FUTAMURA CHEMICAL COMPANY LIMITED) 28 October 2013 (2013-10-28) paragraphs [0001], [0007], [0020], [0024], [0030], [0050], table 1 | 1-3, 5-14, 16 |
| Y | JP 2017-95488 A (MEXICHEM AMANCO HOLDING SOCIEDAD ANONIMA DE CAPITAL BARIABLE) 01 June 2017 (2017-06-01) paragraphs [0009], [0026], [0034], [0037] | 1-3, 5-14, 16 |
| Y | JP 2013-170129 A (MITSUBISHI MATERIALS CORPORATION) 02 September 2013 (2013-09-02) paragraphs [0010], [0026]-[0030] | 1-3, 5-14, 16 |
| Y | US 5411707 A (THE UNITED STATES OF AMERICAN AS REPRESENTED BY THE ADMINISTRATOR OF THE ENVIRONMENTAL PROTECTION AGENCY) 02 May 1995 (1995-05-02) column 2, fourth paragraph | 1-3, 5-14, 16 |

[✓] Further documents are listed in the continuation of Box C.      [✓]  See patent family annex.

| | |
|---|---|
| *    Special categories of cited documents: | "T"   later document published after the international filing date or priority date and not in conflict with the application but cited to understand the principle or theory underlying the invention |
| "A"   document defining the general state of the art which is not considered to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
|---|---|
| **05 November 2021** | **22 November 2021** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**

| International application No. |
| --- |
| **PCT/JP2021/035076** |

**C.    DOCUMENTS CONSIDERED TO BE RELEVANT**

| Category* | Citation of document, with indication, where appropriate, of the relevant passages | Relevant to claim No. |
| --- | --- | --- |
| Y | 前田恒昭, 外, 「揮発性物質のパージアンドトラップGC/MS」について, ぶんせき, 1995, vol. 251, no. 11, pp. 935, 936, (MAEDA, Tsuneaki et al.), non-official translation (About "purge and trap GC / MS of volatile substances". Analysis.) p. 935, right column, second paragraph, p. 936, left column, third paragraph | 1-3, 5-14, 16 |
| Y | JP 2003-344378 A (EBARA CORPORATION) 03 December 2003 (2003-12-03) paragraphs [0001], [0023]-[0025], [0033] | 7-14, 16 |
| A | JP 2019-155215 A (OSAKA GAS CHEMICALS KABUSHIKI KAISHA) 19 September 2019 (2019-09-19) paragraphs [0047], [0050], table 1 | 4, 15 |
| A | JP 2008-55318 A (OSAKA GAS CHEMICALS KABUSHIKI KAISHA) 13 March 2008 (2008-03-13) entire text, all drawings | 1-16 |
| A | JP 2000-346759 A (DKK CORPORATION) 15 December 2000 (2000-12-15) entire text, all drawings | 1-16 |

Form PCT/ISA/210 (second sheet) (January 2015)

INTERNATIONAL SEARCH REPORT
Information on patent family members

International application No.

**PCT/JP2021/035076**

| Patent document cited in search report | | | Publication date (day/month/year) | Patent family member(s) | | Publication date (day/month/year) |
|---|---|---|---|---|---|---|
| JP | 2013-220413 | A | 28 October 2013 | KR 10-2013-0118248 | A | |
| JP | 2017-95488 | A | 01 June 2017 | US 2012/0203037 A1 paragraphs [0009], [0026], [0034], [0037] WO 2011/045559 A1 EP 2952499 A1 KR 10-2012-0083909 A CN 102695692 A | | |
| JP | 2013-170129 | A | 02 September 2013 | (Family: none) | | |
| US | 5411707 | A | 02 May 1995 | (Family: none) | | |
| JP | 2003-344378 | A | 03 December 2003 | (Family: none) | | |
| JP | 2019-155215 | A | 19 September 2019 | (Family: none) | | |
| JP | 2008-55318 | A | 13 March 2008 | (Family: none) | | |
| JP | 2000-346759 | A | 15 December 2000 | (Family: none) | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012101159 A